Europäisches Patentamt

European Patent Office

Office européen des brevets

(19)

(11) **EP 0 862 590 B1**

(12) **EUROPÄISCHE PATENTSCHRIFT**

(45) Veröffentlichungstag und Bekanntmachung des Hinweises auf die Patenterteilung:
**18.08.1999 Patentblatt 1999/33**

(21) Anmeldenummer: **96939094.7**

(22) Anmeldetag: **18.11.1996**

(51) Int Cl.[6]: **C08F 220/06**, A61L 15/60

(86) Internationale Anmeldenummer:
**PCT/EP96/05075**

(87) Internationale Veröffentlichungsnummer:
**WO 97/19116 (29.05.1997 Gazette 1997/23)**

(54) **FLÜSSIGKEITSABSORBIERENDE POLYMERE, VERFAHREN ZU DEREN HERSTELLUNG UND DEREN VERWENDUNG**

FLUID-ABSORBING POLYMERS, PROCESS FOR THE PREPARATION AND USE THEREOF

POLYMERES ABSORBANT DES LIQUIDES, PROCEDE DE PREPARATION ET UTILISATION

(84) Benannte Vertragsstaaten:
**AT BE CH DE DK ES FI FR GB GR IE IT LI LU MC NL PT SE**

(30) Priorität: **21.11.1995 DE 19543369**
**11.11.1996 DE 19646484**

(43) Veröffentlichungstag der Anmeldung:
**09.09.1998 Patentblatt 1998/37**

(73) Patentinhaber: **STOCKHAUSEN GmbH & CO. KG**
**47805 Krefeld (DE)**

(72) Erfinder:
• **STOCKHAUSEN, Dolf**
**D-47800 Krefeld (DE)**
• **HARTAN, Hans-Georg**
**D-47625 Kevelaer (DE)**
• **BREHM, Helmut**
**D-47800 Krefeld (DE)**
• **JONAS, Gerd**
**D-47906 Kempen (DE)**
• **MESSNER, Bernfried**
**Greensboro, NC 27408 (US)**
• **PFLÜGER, Klaus**
**D-47807 Krefeld (DE)**

(74) Vertreter: **Wolff, Felix, Dr. et al**
**Kutzenberger & Wolff**
**Theodor-Heuss-Ring 23**
**50668 Köln (DE)**

(56) Entgegenhaltungen:
**EP-A- 0 280 541**          **EP-A- 0 522 570**
**WO-A-93/21237**          **WO-A-95/17455**
**US-A- 5 408 019**

## Beschreibung

Die Erfindung betrifft superabsorbierende Polymere für wäßrige Flüssigkeiten, Verfahren zu deren Herstellung und deren Verwendung. Die Polymerisate auf Basis von Carboxylatgruppen enthaltenden Monomeren, erhalten durch eine spezielle Kombination aus Vernetzern und weiteren Comonomeren, zeigen eine bisher nicht erreichte Eigenschaftskombination in Bezug auf Aufnahmegeschwindigkeit, hohe Retention bei hoher Absorption unter Druck, niedrigen löslichen Anteilen und guter Permeabilität der Gelschicht für wäßrige Flüssigkeiten unter Druckbelastung und stabiler Oberflächenvernetzung.

Superabsorbierende Polymere sind wasserunlösliche, vernetzte Polyrnere, die in der Lage sind, unter Quellung und Ausbildung von Hydrogelen große Mengen an wäßrigen Flüssigkeiten und Körperflüssigkeiten, wie z.B. Urin oder Blut, aufzunehmen und die absorbierte Flüssigkeitsmenge unter einem bestimmten Druck zurückzuhalten. Durch diese charakteristischen Absorptionseigenschaften finden die Polymeren hauptsächlich Anwendung bei der Einarbeitung in Sanitärartikel, wie z.B. in Babywindeln und Damenbinden.

Bei den kommerziell verfügbaren superabsorbierenden Polymeren handelt es sich im wesentlichen um vernetzte Polyacrylsäuren oder vernetzte Stärke/Acrylsäure-Pfropfcopolymerisate, bei denen die Carboxylgruppen teilweise mit Natrium- oder Kalium-Ionen neutralisiert sind.

Die Herstellung superabsorbierender Polymere erfolgt überwiegend durch Polymerisation wäßriger Lösungen von Mischungen aus teilneutralisierter Acrylsäure und Vernetzer zu einem Polymergel, das nach mechanischer Zerkleinerung getrocknet und auf eine bestimmte Korngröße gemahlen wird. Alternativ können Polymerpulver auch über eine inverse Suspensionspolymerisation gewonnen werden, bei der die wäßrige Monomerphase in einer Ölphase, die z.B. aus Cyclohexan besteht, mit Hilfsmitteln suspendiert und anschließend polymerisiert wird. Durch azeotrope Destillation entfernt man das sich in den Polymertröpfchen befindende Wasser und isoliert anschließend die Polymerisatteilchen durch Abfiltrieren von der Ölphase.

Im Zuge der technischen Weiterentwicklung der superabsorbierenden Polymere hat sich das Anforderungsprofil an diese Produkte über die Jahre deutlich verändert. Während in der Entwicklung der Superabsorber zunächst allein das sehr hohe Quellvermögen bei Kontakt mit Flüssigkeit im Vordergrund stand, hat sich später gezeigt, daß es nicht nur auf die Menge der absorbierenden Flüssigkeit ankommt, sondern auch auf die Festigkeit des gequollenen Gels. Retention einerseits und Gelfestigkeit andererseits stellen jedoch gegenläufige Eigenschaften dar, wie bereits aus der US 3 247 171 und der US Re 32,649 bekannt ist. Das bedeutet, daß Polymere mit besonders hoher Retention nur eine geringe Festigkeit des gequollenen Gels aufweisen mit der Folge, daß das Gel unter einem angewendeten Druck (z.B. Körperdruck) deformierbar ist und die weitere Flüssigkeitsaufnahme verhindert. Es muß also ein ausgewogenes Verhältnis von Retention und Gelstärke angestrebt werden, damit im Anwendungsfall die Flüssigkeitsaufnahme auch gegen einen ausgeübten Druck erfolgen kann. Diese spezifische Absorptionseigenschaft wird in der EP 339 461 als Aufnahme unter Druck bezeichnet.

Die Methode zur Messung der Flüssigkeitsaufnahme unter Druck (AUL) wird bei verschieden hohen Belastungen durchgeführt. Im Zuge der gestiegenen Anforderungen an Superabsorber hat es sich herausgestellt, daß die ursprüngliche Prüfbelastung von 21 g/cm$^2$ (0,3 psi) nicht mehr den erwünschten Eigenschaftsstandard mißt, wie er für Inkontinenzprodukte bzw.für Windelkonstruktionen mit niedrigen Fluffgehalten und hohen Mengen Superabsorber erforderlich ist. Demzufolge werden heute Druckbelastungen bei 42 g/cm$^2$ (0.6 psi) und vorzugsweise bei 63 g/cm$^2$ (0.9 psi) gemessen.

Der zunehmenden Tendenz, die Sanitärartikel immer kleiner und dünner zu gestalten, kann nur dadurch entsprochen werden, daß man den großvolumigen Fluffanteil in der Windel reduziert und gleichzeitig den Anteil an Superabsorber erhöht. Hierdurch muß der Superabsorber zusätzliche Aufgaben bezüglich Flüssigkeitsaufnahme- und transport übernehmen, die vorher der Fluff erfüllte.

Die Superabsorber können durch das Verfahren der nachträglichen Oberflächenvernetzung in Ihrem Eigenschaftsprofil verbessert werden, insbesondere auch in ihrer Flüssigkeitsaufnahme unter Druck, da das bekannte Phänomen des "gel blocking" unterdrückt wird, bei dem angequollene Polymerteilchen miteinander verkleben und eine weitere Flüssigkeitsaufnahme und Flüssigkeitsverteilung in der Windel behindern. Während der Nachvernetzung werden die Carboxylgruppen der Polymermoleküle an der Oberfläche der Superabsorberteilchen mit Vernetzungsmitteln unter erhöhter Temperatur vernetzt. Als Vernetzer finden u.a. mehrwertige Metallsalze, Glycidylverbindungen, Polyole, Polyepoxide, Polyamine, Alkylencarbonate und Polyethylenglykole Verwendung. Der Schritt der Nachvernetzung kann auch mehrfach erfolgen. Aus den Patentschriften geht hervor, daß die erhöhte Flüssigkeitsaufnahme unter Druck mit dem Nachteil einer deutlichen Reduzierung der Retention verbunden ist. Es besteht demnach ein Bedarf an Superabsorbervorprodukten, die bei der Nachvernetzung einen weniger starken Abfall in den Retentionswerten aufweisen. Weiterhin ist es bisher nicht gelungen, die Oberflächenvernetzung dauerhaft am Polymergerüst zu fixieren, so daß ihre Effekte durch mechanische Einwirkungen auf den Absorber zum größten Teil wieder zunichte gemacht werden. Für die Verarbeitung der Superabsorber werden je nach Anwendungsfall unterschiedliche Siebfraktionen eingesetzt, so z.B. für Windeln zwischen 100 und 800μm, für Kabelisolierungen unter 200μ. D.h. im Falle der Anwendung in Kabeln sind die Feinanteile der Superabsorber wegen ihrer Tendenz zum gel blocking von Vorteil, da dadurch das in das Kabel eindringende Wasser abgeblockt wird. In der Windel ist dieser Effekt unerwünscht, da er die Flüssigkeitsaufnahme und Verteilung behindert, deshalb wählt man gröbere Siebfraktionen aus.

Neben einem hohen Niveau der Retention und der Flüssigkeitsaufnahme unter Druck müssen Superabsorber niedrige Mengen an löslichen Anteilen enthalten, die aufgrund unvollkommener Vernetzung während der Polymerisationsreaktion auftreten und im Anwendungfall nicht vollständig im Polymerkörper zurückgehalten werden. Dies führt letztlich zu einer Verminderung der Fähigkeit des Superabsorbers zur Flüssigkeitsaufnahme und Flüssigkeitsverteilung in der Windel. Als Grenzwerte für niedrige lösliche Anteile werden z.B. in der US Re. 32,649 7.5% nach 1 Stunde und 17% nach 16 Stunden angegeben. Gemessen an den heute von den Hygieneartikel herstellenden Produzenten gewünschten Produkteigenschaften liegen diese Grenzwerte für die löslichen Anteile viel zu hoch.

Die Optimierung der Gebrauchseigenschaften der superabsorbierenden Polymere erfolgte in der Vergangenheit vor allem durch Variation der Vernetzerart und Vernetzermenge, durch den pH-Wert während der Polymerisation und durch Nachbehandlung der Polymerpartikel im Sinne einer Beschichtung oder Oberflächennachvernetzung. Bislang konnten jedoch noch keine Superabsorber zur Verfügung gestellt werden, die die Eigenschaftskombinationen aus hoher Retention, hohem AUL ($63 \text{ g/cm}^2$, 0.9psi) und niedrigen löslichen Anteilen bei gleichzeitig hoher Permeabilität in der Gelschicht für wäßrige Flüssigkeiten unter Druckbelastung, hoher Aufnahmegeschwindigkeit und nachhaltiger Oberflächenvernetzung in sich vereinen.

Der WO 94/09043 liegt die Aufgabestellung zugrunde, neue superabsorbierende Polymere mit einem für wäßrige Flüssigkeiten erhöhten Aufnahmevermögen, auch unter Druckbelastung bereitzustellen. Sie beschreibt als Lösung dieser Aufgabe doppelt vernetzte Superabsorber, deren Herstellung in der ersten Stufe die Vernetzung während der Polymerisation mit Methylenbisacrylamid, Bis(acrylarnido)essigsäure, Allylacrylat, Allylmethacrylat, Estern bzw. Amiden mit endständigen Vinyl- und Allyl-Funktionen oder hoch ethoxyliertem Trimethylolpropantriacrylat vorsieht und in einer zweiten Stufe die entstandenen Polymerpartikel an der Oberfläche mit einem Vernetzer beschichtet und dann vernetzt. Bei diesem, an sich bereits bekannten Verfahren, sind die bevorzugten Oberflächenvernetzer Polyhydroxyverbindungen, die zusammen mit Wasser oder Wasser/Lösemittelgemischen aufgebracht und bei erhöhten Temperaturen (175 - 230°C) zur Reaktion gebracht werden, nachdem zuvor die Feuchtigkeit des Polymergels der ersten Stufe zumindest teilweise entfernt wurde.

Durch die Kombination eines der genannten primären Vernetzer mit den sekundären Oberflächenvernetzern sollen angeblich einzigartige Produkteigenschaften bezüglich der Retention und der Flüssigkeitsaufnahme unter Druck erreicht werden, wodurch die vorteilhafte Anwendung in Hygieneartikeln, bei denen die absorbierenden Polymere beträchtliche Mengen an Flüssigkeit aufsaugen und auch unter Druckbelastung zurückhalten müssen, gegeben ist. Bei der Durchsicht der Versuchsergebnisse fällt auf, daß die Polymere allein durch die Lagerung bei hoher Temperatur ohne Zusatz eines Nachvernetzers einen deutlichen Eigenschaftssprung machen. Durch die Nachvernetzung erfolgt dann nochmals eine Anhebung der Flüssigkeitsaufnahme unter Druck bei $42 \text{ g/cm}^2$ ( 0.6 psi ). Diese AUL-Werte liegen dann je nach Ausführungsbeispiel in einem Bereich von 10 bis 26 g/g, wobei die Produkte mit den höchsten AUL-Werten aber nur Absorptionswerte von 30 g/g erreichen. Absorptionswerte von über 30 g/g sind, wie das Beispiel 3B (Absorption 35 g/g und AUL 16.5 g/g) zeigt, nur aufKosten des AUL bei $42 \text{ g/cm}^2$ (0.6psi) möglich. Damit wird deutlich, daß die Produkte mit der speziellen Vernetzerkombination der WO 94/09043 keinesfalls den hohen Anforderungen genügen, die heutzutage an derartige vernetzte Polymerisate gestellt werden, d.h. die dort für $42 \text{ g/cm}^2$ (0.6 psi) gemessenen Spitzenwerte werden heute für eine höhere Belastung bei $63 \text{ g/cm}^2$ (0.9 psi), kombiniert mit Absorptionswerten von deutlich über 30 g/g gefordert.

Die WO 93/21237 beschreibt superabsorbierende Polymere, die mit ungesättigten Estern von Polyalkylglykolen vernetzt sind und die in einem Nacherhitzungsprozeß eine Eigenschaftsverbesserung bezüglich Retention und Flüssigkeitsaufnahme unter niedrigem Druck $21 \text{ g/cm}^2$ ( 0.3 psi ) auf jeweils 25 g/g erlangen. Ethoxyliertes Trimethylolpropantriacrylat ist der bevorzugte Vernetzer, wobei die Anzahl der EO-Einheiten pro Polyglykolkette zwischen 2 und 7 liegen kann. Gemäß den Ausführungen in dieser Schrift führt die Verwendung von nicht oder nur gering ethoxyliertem Trimethylolpropantriacrylat zu wesentlich schlechteren Eigenschaften der damit vernetzten Superabsorber. In Analogie zu den Polymeren der WO 94/09043 erfüllen auch die hier beschriebenen Produkte nicht die heutzutage gestellten Anforderungen bezüglich der Absorption unter höherem Druck bei $63 \text{ g/cm}^2$ (0.9 psi). In der Abbildung 13 auf Seite 8/8 der WO 93/21237, die den Verlauf der Flüssigkeitsaufnahme unter Druck für verschiedene Druckbelastungen darstellt, zeigt sich ganz klar die Schwäche der dort beschriebenen Polymere, deren Meßwerte von ca. 18 g/g im interessanten Druckbelastungsbereich von $63 \text{ g/cm}^2$ (0.9 psi) völlig unbefriedigend sind. Dies gilt um so mehr, als die Meßwerte an einer völlig unüblichen Siebfraktion von 300 - 600 µm ermittelt wurden, die per se höhere Meßwerte liefert, als die praxisübliche Siebfraktion von 150 - 800 µm.

Der US Re. 32,649 ist die Herstellung nicht gepfropfter superabsorbierender Polymere mit hohem Gelvolumen, hoher Gelstärke, gemessen über den Schermodul und niedrigen löslichen Anteilen zu entnehmen. Das Gelvolumen soll mindestens 20 g/g betragen und der Höchstwert der löslichen Anteile, gemessen nach 1 Stunde soll 7.5 % nicht überschreiten und im Gleichgewichtszustand nach 16 Stunden maximal 17 % betragen. Die Polymere werden bevorzugt in einer niedrigen wäßrigen Ansatzkonzentration von 8 bis 24 Gew.% aus nicht neutralisierter Acrylsäure aufgebaut, die Vernetzung erfolgt vorzugsweise durch N,N'-Methylenbisacrylamid, Trimethylolpropantriacrylat oder Triallylamin. Nachfolgend wird zerkleinert, neutralisiert, gemahlen und getrocknet. Das Herstellungsverfahren der Re 32,649 weist wesentliche Verfahrensmängel auf. Zum einen liegt aufgrund der niedrigen Ansatzkonzentration und einer mehrstündigen Nacherhitzung des Polymergels eine unwirtschaftlich niedrige Raum/Zeit-Ausbeute vor, zum anderen ist der Verfahrensschritt der nachträglichen Neutralisation des festen Polymergels technisch sehr zeitaufwendig und nicht in der Qualität durchzuführen, wie es eine Neutralisation in der vorausgehenden Lösung sein kann. Nachträglich neutralisierte Polymergele sind in der Regel nicht gleichmäßig durchneutralisiert und oftmals aufgrund von ungleichmäßiger Alkaliverteilung verfärbt. Als Folge der ungleichmäßigen Neutralisation können auch starke Qualitätsschwankungen innerhalb des Produktes entstehen.

Die EP 690 077 beschreibt Superabsorber mit verbesserter Bruchfestigkeit durch Verwendung von Polyethylenglycolen und Derivaten wie z.B. Methoxypolyethylenglycol-Methacrylaten als Additive oder Comonomere. Die Superabsorber zeichnen sich dadurch aus, daß bei einem Mahltest im Verhältnis zu einem Referenzprodukt weniger Partikel im Bereich < 5 µm entstehen. Aussagen zu den Quelleigenschaften der Superabsorber nach der mechanischen Belastung werden nicht gemacht. Die beschriebenen Superabsorber sind weder mit speziellen Vorvernetzern noch mit Oberflächen-Nachvernetern hergestellt. Die angegebenen Absorptionen unter Druck von mindestens 17 g/g, bevorzugt > 20 g/g (AUL) beziehen sich lediglich auf eine äußerst niedrig gewählte Belastung von ca. 20 g/cm$^2$ und liegen, gemessen am Stand der Technik, nicht in einem diskutablen Bereich. Superabsorber des Standes der Technik bieten seit geraumer Zeit höhere Absorptionen unter höherem Druck (63 g/cm$^2$) von > 20 g/g.

Die Verwendung von Polyethylenglycolen zur Entstaubung von Superabsorbern ist hingegen bekannt:
In der WO 94/22940 werden superabsorbierende Polymere mit einem geringen Staubanteil beschrieben, die aufgrund einer Oberflächenbeschichtung mit Polyol oder Polyethylenglykol entstehen und die eine Flüssigkeitsaufnahme unter niedrigem Druck bei 21 g/cm$^2$ ( 0.3 psi ) von >20g/g aufweisen. Die Behandlung von Superabsorbern mit derartigen Substanzen ist bereits in der WO 93/21237 vorbeschrieben (Seite 12, Z.15). Diese abriebverbesserte, staubreduzierte Einstellung erfordert im Herstellungsprozeß der superabsorbierenden Polymere allerdings einen zusätzlichen Verfahrensschritt.

Aus dem Stand der Technik ist bisher kein Vorschlag zur Herstellung von Superabsorbem mit der gewünschten Eigenschaftskombinationen aus hoher Permeabilität für wäßrige Flüssigkeiten, stabiler Oberflächenvernetzung, hoher Retention, hoher Aufnahmegeschwindigkeit, druckfestem Hydrogel und niedrigem löslichen Anteil zu entnehmen.

Niedrige lösliche Anteile sind letztlich auch eine Voraussetzung für Superabsorber, die einen anwendungsgemäßen Quelldruck über längere Zeit aufweisen müssen. Liegen die löslichen Anteile über 12 %, kann der Superabsorber nach einer Standzeit von 16 h unter Druckbelastung, etwa in einer Windel, nicht mehr die anwendungstechnisch geforderte Flüssigkeitsmenge speichern. Insbesondere die Permeabilität in der Schicht (d.h. in x,y-Richtung) des absorbierenden Artikels, die den Superabsorber enthält, gewinnt zunehmend an Bedeutung, wenn Konstruktionen mit immer geringerer Fluffmenge bei gleichzeitig erhöhter Superabsorbermenge eingesetzt werden. Dies gewinnt zusätzlich an Bedeutung, wenn die Flüssigkeitsverteilung unter Druck von z.B. 50 g/cm$^2$ erfolgen muß.
Eine geeignete Methode zur Bestimmung der Permeabilität einer Gelschicht in x,y-Richtung unter Druck ist die Methode der Bestimmung der Absorption unter Druck durch eine flächenreduzierte Öffnung im Boden einer sogenannten AAP-Zylindereinheit.

Die AAP-Zylindereinheit wurde in EP 640 330 auf Seite 14 (Absorption against Pressure test) beschrieben. Superabsorbierende Polymere, die dem heutigen Stand der Technik bezüglich der Konstruktion von absorbierenden Artikeln genügen sollen, müssen den Flüssigkeitstransport in x,y Richtung, d. h. innerhalb der Gelschicht dergestalt unterstützen, daß bei der genannten Meßmethode möglichst hohe Absorptionswerte erreicht werden. Die gemessenen Werte werden dann mit der jeweiligen Absorption gegen Druck bei gleicher Belastung (20 g/cm$^2$ bzw. 50 g/cm$^2$) verrechnet und als prozentuale Absorption angegeben. Das Anforderungsprofil an größere Mengen Superabsorber enthaltende Windeln erfordert Werte im Bereich > 50 %, wobei für den flächenreduzierten AAP-Wert mindestens 15 g/g erreicht werden müssen.

Es bestand daher die Aufgabe neue Polymerisate und ein Verfahren zu deren Herstellung zur Verfügung zu stellen, die als Superabsorber zur Verwendung in Windelkonstruktionen oder bei anderen technischen Anwendungen eine gegenüber dem Stand der Technik verbesserte Kombination der Eigenschaften aus Retention, Flüssigkeitsaufnahme unter Druck bei 63 g/cm$^2$ (0.9 psi) und löslichen Anteilen aufweisen und die aufgrund ihrer Polymerzusammensetzung eine effiziente und nachhaltige Oberflächenvernetzung besitzen und aufgrund ihrer Permeabilität auch unter Druck (50g/cm$^2$) eine gute Flüssigkeitsverteilung im gequollenen Zustand erlauben. Darüber hinaus sollen die Superabsorber eine hohe Sauggeschwindigkeit und einen hohen Quelldruck aufweisen.

Es wurde nun überraschenderweise gefunden, daß wäßrige Flüssigkeiten absorbierende vernetzte Polymerisate, aufgebaut aus teilneutralisierten, monoethylenisch ungesättigten Säuregruppen tragenden Monomeren, gegebenenfalls weiteren, damit copolymerisierbaren Monomeren sowie gegebenenfalls als Pfropfgrundlage geeigneten wasserlöslichen Polymeren, die unter Verwendung einer Vernetzer-Kombination aus

I.     $CH_2=CR^6-CO(OCHR^3-CHR^3)_zO-CH_2-CR^6=CH_2$

II.     $CH_2=CR^6-R^5-(OCHR^3-CHR^3)_vOR^4$

III.     $R^1-[O(CHR^3-CHR^3O)_u-CO-R^2]_x,$

und/oder Di- bzw. Triallylamin und/oder Bisacrylamide
mit

$R^1$ : mehrwertiges C2-10-Alkyl,
$R^2$: linear oder verzweigt C2-10-Alkenyl,
$R^3$: H, $CH_3$, $C_2H_5$,
$R^4$: H, linear oder verzweigt C1-10-Alkyl,
$R^5$: CO, $CH_2$,
$R^6$: H, $CH_3$,
x: 2-6,
u: 0-15,
v: 1-45,
z: 3-20

hergestellt und anschließend einer Oberflächenvernetzung unterzogen werden, eine Retention größer 30g/g und eine Flüssigkeitsaufnahme unter Druck bei 63 g/cm² (AUL 0.9 psi) größer 20 g/g aufweisen und deren lösliche Anteile nach 1 Stunde kleiner 6,5 % und nach 16 Stunden kleiner 10 % sind und deren Permeabilität innerhalb der Gelschicht die Nutzung von mindestens 50 %, -Test (Absorption gegen Druck bei auf x% reduzierter Saugfläche) bei mindestens 15 g/g, vorzugsweise mindestens 20 g/g AAP50-$A_x$ erlaubt. Aufgrund der erfindungsgemäßen vorzugsweise mindestens 60 % der Absorptionskapazität unter einer Belastung von 50 g/cm² im AAP-$A_x$ Polymerstruktur findet eine verbesserte und nachhaltige Vernetzung zwischen dem Polymer und dem Oberflächenvernetzer statt. Dies äußert sich darin, daß der AUL (63 g/cm²) nach einem Belastungstest zur Ermittlung der Stabilität der Oberflächenvernetzung noch Werte größer 18 g/g aufweist, was auf eine ungewöhnlich stabile Oberflächenvernetzung und eine äußerst gründliche Reaktion mit dem Oberflächenvernetzer schließen läßt.

Die erfindungsgemäß zu verwendenden Vernetzer gemäß Formel I enthalten eine (Meth)allylfunktion und eine (Meth)acrylsäureesterfunktion und eine zwischen diesen beiden Funktionen angeordnete hydrophile Kette, die aus mindestens drei, vorzugsweise 5 bis 20 Ethylenoxideinheiten besteht. Die Verwendung von gemischten Ethylenoxid/ Propylenoxidketten, die als Random- oder Blockcopolymer hergestellt sein können, ist möglich. Die Löslichkeit des Vernetzers in der zu polymerisierenden Monomerlösung kann durch das Verhältnis von EO/PO-Einheiten eingestellt werden. Die Vernetzer lassen sich zum Beispiel durch Veresterung von alkoxyliertem Allylalkohol mit (Meth)acrylsäure herstellen. In den erfindungsgemäßen Vernetzern können sich durch das Herstellverfahren bedingte Restgehalte von Ausgangskomponenten befinden, die sich jedoch auf die Eigenschaften der Superabsorber nicht nachteilig auswirken. Die Vernetzer gemäß der Formel I oder deren Gemische werden zu 0-1,0 Gew.% und bevorzugt zu 0,05-0,6 Gew.% und besonders bevorzugt 0,1-0,4 Gew.% bezogen auf die gesamten Monomeren eingesetzt.

Die erfindungsgemäß mit den Vernetzern zu verwendenen Monomere gemäß Formel II stellen bevorzugt (Meth)acrylsäureester oder (Meth)allylalkoholether von Polyalkylenglykolen dar. In einer bevorzugten Ausführungsform ist die Polyoxyalkylenglykolkette endständig mit einem Alkylrest versehen. Die C-Anzahl im Alkylrest liegt im Bereich 1-10, bevorzugt im Bereich 1-7 und besonders bevorzugt 1-4. Bei der Auswahl der Alkylgruppe ist auch auf den tensidischen Charakter des Monomers zu achten und gegebenenfalls muß zur Vermeidung von Schaumbildung, wie sie in einigen Polymerisationsverfahren durchaus störend auftreten kann, der Alkylrest entsprechend angepaßt werden. Die Polyoxyalkylenglykolkette setzt sich vorzugsweise aus Ethylenoxid und/oder Propylenoxid zusammen, wobei die Löslichkeit des Vernetzers in der wäßrigen Monomerlösung durch das Verhältnis von EO/PO eingestellt werden kann. Der Gehalt an Alkylglykoleinheiten in der Kette liegt im Bereich 1-45, bevorzugt im Bereich 1-30 und besonders bevorzugt im Bereich 5-25. Die Monomere gemäß der Formel II bzw. deren Gemische werden zu 0,1-10 Gew.% und bevorzugt zu 0,5-5 Gew.% und besonders bevorzugt zu 1,0-3,5 Gew.% bezogen auf die gesamten Monomeren eingesetzt. Die Monomeren nach II sind im Handel erhältlich, z.B. die Methylpolyethylenglykolmethacrylate von der Firma Interorgana unter der Bezeichnung Bisomer MPEG(x)MA (x=350, 1000, 2000).

Die erfindungsgemäß zu verwendenden Vernetzer gemäß III stellen Ester von Polyhydroxyverbindungen mit ungesättigten Carbonsäuren dar, die in einer bevorzugten Ausführungsform alkoxyliert sind. Bevorzugt werden C3-6-Polyhydroxyverbindungen mit 2-4 Hydroxylgruppen als Ausgangsverbindungen für die Synthese derartiger Vernetzer, beispielsweise Trimethylolpropan, Glycerin, Pentaerythrit, 1,3-Propandiol, Propylenglykol oder 1,4-Butandiol. Findet vor der Veresterung eine Alkoxylierung des Alkohols statt, so wird bevorzugt Ethylenoxid eingesetzt. Bevorzugt werden alkoxylierte Polyhydroxyverbindungen ab u=1, besonders bevorzugt ab u=3 eingesetzt. Als saure Komponente wird bevorzugt (Meth)acrylsäure verwendet. In einer weiteren bevorzugten Ausführungsform des Vernetzers III wird Polyethylenglykol-di(meth)acrylat verwendet. In einer besonders bevorzugten Ausführungsform des Vernetzers III werden Di- bzw. Triallylamin und/oder N,N-Methylenbisacrylamid und/oder Bisacrylamidoessigsäure verwendet. Auch Mischungen der zuletzt genannten Vernetzer mit den zuvor genannten Carbonsäureestern der Polyhydroxyverbindungen zeigen eine ausgezeichnete Wirksamkeit.

Die Vernetzer gemäß III bzw. deren Gemische werden zu 0,01-1,0 Gew.% und bevorzugt zu 0,05-0,6 Gew.% und besonders bevorzugt zu 0,05-0,3 Gew.% bezogen auf die Monomeren eingesetzt.

Die erfindungsgemäßen Vernetzer nach III sind teilweise im Handel erhältlich, z.B. Trimethylolpropan-oxethylat-triacrylat von der Fa. Cray Valley unter der Bezeichnung Sartomer SR 415 (20EO), Craynor 435 (15EO), Sartomer RO 208 (9EO), Sartomer 454 (3EO) bzw. Pentaerythrit-oxethylat-tetraacrylat unter der Bezeichnung Craynor SR 494 (5EO) und Servocure RTT 192 (5EO) von der Fa. Servo Delden BV, Glycerin-ethoxylat-triacrylat (5,5EO) unter der Bezeichnung Sartomer 921 und Glycerin-propoxylat-triacrylat unter der Bezeichnung Sartomer 9021 von der Fa. Cray Valley sowie Polyethylenglykol-400-diacrylat als Craynor SR 344 bzw. Polyethylenglykol-600-dimethacrylat als Craynor SR 252 von der Fa. Cray Valley.

Überraschenderweise hat es sich herausgestellt, daß durch die erfindungsgemäße Vernetzer/Monomer-Kombination eine gegenseitige Solubilisierung von in der wäßrigen Monomerlösung löslichen und unlöslichen Vernetzerbestandteilen möglich ist. Dadurch wird auch die Verwendung von Vernetzem möglich, die normalerweise aufgrund ihrer mangelnden Löslichkeit nicht oder nur in äußerst begrenzter Menge einsetzbar sind.

In einer weiteren bevorzugten Ausführungsform hat sich die Verwendung von Mischungen aus hoch und niedrig alkoxylierten Vernetzern/Monomeren gemäß I, II und III bei der Vernetzung der erfindungsgemäßen Superabsorber bewährt.

Das erfindungsgemäß zu verwendende, wäßrige Flüssigkeiten absorbierende Polymerisat wird erhalten durch Polymerisation von ethylenisch ungesättigten, Säuregruppen tragenden Monomeren, beispielsweise aus Acrylsäure, Methacrylsäure, Vinylessigsäure, Maleinsäure, 2-Acrylamido-2-methylpropansulfonsäure, Vinylsulfonsäure, (Meth)allylsulfonsäure bzw. deren Gemische in Gegenwart der Vernetzer/Monomer-Kombination aus den Komponenten I, II und III. Der Anteil an diesen sauren Monomeren beträgt in der Monomermischung 55 - 99 Gew.%.

Die sauren Monomeren sind mindestens zu 25 Mol% und bevorzugt zu mindestens 50 Mol.% und besonders bevorzugt zu 50 bis 80 Mol % neutralisiert und liegen dann beispielsweise als Natrium-, Kalium- oder Ammoniumsalz bzw. deren Gemische vor. Die Neutralisation wird entweder durch die Zugabe der entsprechenden Alkali- bzw. Ammoniumhydroxyde oder mit den entsprechenden Carbonaten oder Hydrogencarbonaten ausgeführt.

Optional können die erfindungsgemäßen Polymerisate weitere, mit den Säuregruppen tragenden Monomeren copolymerisierbare Comonomere zur Modifizierung der Eigenschaften enthalten. Solche Comonomere können beispielsweise (Meth)acrylamid, (Meth)acrylnitril, Vinylpyrrolidon, Vinylacetamid, Hydroxyethylacrylat, Alkylaminoalkyl (meth)acrylate, Alkylaminopropylacrylamide, Acrylamidopropyltrimethylammoniumchlorid oder deren Gemische sein. Derartige Comonomere sollten einen Anteil von 40 Gew.% nicht überschreiten, da sie die Quellfähigkeit des Superabsorbers gegebenenfalls beeinträchtigen können.

Die erfindungsgemäßen Polymerisate können wasserlösliche Polymere als Pfropfgrundlage in Mengen bis zu 30 Gew.% enthalten. Dazu zählen unter anderem teil- oder vollverseifte Polyvinylalkohole, Stärke oder Stärkederivate, Cellulose oder Cellulosederivate, Polyacrylsäuren, Polyglykole, Polysaccharide oder deren Gemische. Die Molekulargewichte der als Pfropfgrundlage zugesetzten Polymere müssen an die Gegebenheiten der Polymerisationsbedingungen angepaßt sein. So kann es z.B. im Falle einer wäßrigen Lösungspolymerisation aus Gründen der Viskosität der Polymerisatlösung erforderlich sein, nur niedrig- oder mittelmolekulare Polymere einzusetzen, wohingegen bei der Suspensionspolymerisation dieser Faktor eine untergeordnete Rolle spielt.

Neben Polymerisaten, die durch vernetzende Polymerisation teilneutralisierter Acrylsäure zu erhalten sind, werden bevorzugt solche verwendet, die zusätzliche Anteile von pfropfpolymerisierter Stärke oder von Polyvinylalkohol enthalten.

Die Herstellung der erfindungsgemäßen Superabsorber geschieht prinzipiell nach zwei Methoden:

Nach der ersten Methode wird die teilneutralisierte Acrylsäure in wäßriger Lösung in Gegenwart der Vernetzer/Monomerkombination aus I, II und III sowie gegebenenfalls Polymerzusätzen durch radikalische Polymerisation in ein Gel überführt, das dann zerkleinert, getrocknet, gemahlen, nachvernetzt und auf die gewünschte Partikelgröße abgesiebt wird. Die Lösungspolymerisation kann kontinuierlich oder diskontinuierlich durchgeführt werden. Die Patentliteratur weist sowohl ein breites Spektrum an Variationsmöglichkeiten hinsichtlich der Konzentrationsverhältnisse, Temperaturen, Art und Menge der Initiatoren als auch eine Vielzahl von Nachvernetzungsmöglichkeiten aus. Typische Verfahren sind in den folgenden Patentschriften beschrieben. US 4 076 663, US 4 286 082, DE 27 06 135, US 4 076 663, DE 35 03 458, DE 40 20 780, DE 42 44 548, DE 43 23 001, DE 43 33 056, DE 44 18 818.

Die zweite Methode umfaßt das inverse Suspensions- und Emulsionspolymerisationsverfahren. In diesen Prozessen wird eine wäßrige, teilneutralisierte Acrylsäurelösung mit Hilfe von Schutzkolloiden und/oder Emulgatoren in einem hydrophoben, organischen Lösungsmittel dispergiert und durch Radikalinitiatoren die Polymerisation gestartet. Die Vernetzer sind entweder in der Monomerlösung gelöst und werden mit dieser zusammen dosiert oder aber separat und gegebenfalls nachträglich zugefügt. Die Zugabe von gegebenenfalls vorhandenen polymeren Pfropfgrundlagen erfolgt über die Monomerlösung oder durch direkte Vorlage in die Ölphase. Nach Beendigung der Polymerisation wird das Wasser azeotrop aus dem Reaktionsgemisch entfernt und das Polymerprodukt abfiltriert. Eine Oberflächenvernetzung der Polymerteilchen kann sowohl in der Suspension als auch nachträglich am isolierten Polymerpulver vorgenommen werden. Das Verfahrensprinzip ist beispielsweise in den Patentschriften US 43 40 706, DE 37 13 601, DE 28 40 010 beschrieben.

Der Zusatz der Nachvernetzer erfolgt häufig vorteilhafterweise auch in Form einer Lösung in Wasser, organischen Lösemitteln oder deren Mischungen, insbesondere dann, wenn geringe Mengen an Nachvernetzungsmittel angewandt werden. Geeignete Mischaggregate zum Aufbringen des Nachvernetzungsmittels sind z.B. Patterson-Kelley-Mischer, DRAIS-Turbulenzmischer, Lödigemischer, Ruberg-Mischer, Schneckenmischer, Tellermischer und Wirbelschichtmischer, sowie kontinuierlich arbeitende senkrechte Mischer, in denen das Pulver mittels rotierender Messer in schneller Frequenz gemischt wird (Schugi-Mischer). Nachdem der Nachvernetzer mit dem vorvernetzten Polymer vermischt worden ist, wird zur Durchführung der Nachvernetzungsreaktion auf Temperaturen von 120 bis 250 °C, bevorzugt auf 135 bis 200°C und besonders bevorzugt auf 150 bis 185°C erhitzt. Die Zeitdauer der Nacherhitzung ist durch den Punkt begrenzt, bei dem das gewünschte Eigenschaftsprofil des Superabsorbers infolge von Hitzschädigung wieder zerstört wird.

Die erfindungsgemäß hergestellten Superabsorber zeigen eine bisher noch nicht erreichte Kombination von günstigen Eigenschaften. Aufgrund des positiven Einflusses der Vernetzer/Comonomer-Kombination wird die vor der Nachvernetzung vorhandene hohe Retention des Polymers derart gut stabilisiert, daß nach der Oberflächen-Nachvernetzung immer noch eine Retention von über 30 g/g gemessen wird.

Nach erfolgter Flüssigkeitsaufnahme, deren Geschwindigkeit bei kleiner 40 sec, bevorzugt kleiner 35 sec und besonders bevorzugt kleiner 30 sec liegt, zeichnen sich die gequollenen Gelpartikel durch einen trocknen Griff aus, d.h. sie haben nicht die unerwünschte nasse, klebrige Oberfläche, die bei unzureichender Vernetzung/Nachvernetzung entsteht. Hinzu kommt, daß gleichzeitig mit der hohen Retention die löslichen Anteile nach 1 Stunde mit kleiner 6,5%, vorzugsweise kleiner 5 % bzw. nach 16 Stunden mit kleiner 10 %, vorzugsweise kleiner 8 % äußerst niedrig ausfallen. Die Flüssigkeitsaufnahme unter einem Druck (AUL) von 63 g/cm$^2$ (0.9 psi) ist größer 20 g/g, bevorzugt größer 23 g/g und besonders bevorzugt größer 25 g/g.

Aufgrund der erfindungsgemäßen Vernetzer/Monomer-Kombination erhalten die Superabsorber eine ausgezeichnete Durchlässigkeit für Flüssigkeiten im gequollenen Zustand unter Belastung. Die erfindungsgemäße Vernetzer/Monomer-Kombination ermöglicht eine effiziente und nachhaltige Oberflächenvernetzung des Polymerisates. Dies ermöglicht die Herstellung von Superabsorbern, die nach einem Belastungstest zur Überprüfung der Stabilität der Oberflächenvernetzung (SDOV) noch AUL Werte (63 g/cm2) von mindestens 18 g/g, bevorzugt größer 20 g/g und besonders bevorzugt größer 22 g/g aufweisen.

Der Quelldruck der erfindungsgemäßen Polymerisate ist hoch und beträgt nach einer Meßzeit von 20 Minuten mindestens 600 g, bevorzugt mindestens 800 g, und besonders bevorzugt größer 900 g.

Die erfindungsgemäßen hydrophilen Superabsorber finden überall dort ihre Verwendung, wo wäßrige Flüssigkeiten absorbiert werden müssen. Dazu gehören beispielsweise die allgemein bekannten Anwendungen für Superabsorber in konstruktionen zur Aufnahme von Körperflüssigkeiten, wie in Hygieneartikeln in Form von Windeln für Kleinkinder und Inkontinenzprodukten für Erwachsene, in Damenbinden, in Wundpflastern, in Lebensmittelverpackungen, im Agrarbereich bei der Pflanzenaufzucht, in Kabelisolierungen, in absorbierenden Flächengebilden aus Papier, wasserlöslichen Polymeren und thermoplastischen Kunststoffen und Schäumen, sowie als Wirkstoffträger mit der Aufgabe der zeitlich verzögerten Freisetzung an die Umgebung.

Besondere Ausführungsformen der Erfindung sind in den abhängigen Ansprüchen beschrieben.

In den folgenden Beispielen werden die Herstellung und Eigenschaften der erfindungsgemäßen Polymerisate erläutert und in dem Kapitel Prüfmethoden werden die Vorschriften zur Bestimmung der Eigenschaften der Superabsorber beschrieben.

**Prüfmethoden**

**1. Retention**

Die Retention wird nach der in der EP 514 724 (Seite 4, Zeilen 6-22) beschriebenen Methode gemessen.

### 2. Flüssigkeitsaufnahme unter Druck (AUL)

Die Flüssigkeitsaufnahme unter Druck (AUL) wird nach der in der EP 514 724 (Seite 4, Zeilen 23-28) beschriebenen Methode bestimmt. Der AUL 63 wird bei einem Druck von 63 g/cm2 (0,9 psi) gemessen, der AUL 21 (0,3 psi) bei 21 g/cm$^2$.

### 3. Lösliche Anteile (LA)

Die Löslichen Anteile (1h und 16h) werden wie in der US 4 654 039 beschrieben bestimmt, mit der Ausnahme, daß als Testflüssigkeit statt synthetischem Urin eine 0,9%-ige Kochsalzlösung verwendet wird.

### 4. Restmonomere (RM)

Die Restmonomeren (RM) werden aus dem Filtrat von der Bestimmung der löslichen Anteile mittels HPLC-Methode ermittelt und nach dem Verfahren des inneren Standards ausgewertet.

### 5. Quelldruck (QD)

Der Quelldruck wird in einem Stevens-LFRA Texture Analyser (Einstellung: Speed: 1,0 mm/sec; Distance 00, Hold-Stellung) bestimmt. Dazu werden in einem Messzylinder von 7,4 cm Höhe und 2,7 cm Durchmesser 0,500 g des Pulvers (Kornfraktion 300 - 600 mm) eingewogen und mit 10 ml 0,9%-iger Kochsalzlösung versetzt. Sodann wird der Messzylinder(Höhe 3,5 cm, Durchmesser 2,5 cm) in den Zylinder so eingefahren, bis der Stand der Unterkante des zylindrischen Meßkörpers von der Oberfläche der sich im Meßzylinder befindlichen Probe 12 mm beträgt. Durch die Ausdehnung des Gels wird der Meßzylinder nach oben gegen eine Zwei-Weg-Kraftmeßzelle gedrückt und am Gerät in Gramm angezeigt. Der Quelldruck QD wird nach verschiedenen Zeiten gemessen.

### 6. Absorption gegen Druck bei reduzierter Saugfläche ( AAP- A$_x$)

Mit Hilfe dieses Tests wird die Fähigkeit eines Superabsorbers, unter einem definierten Druck Flüssigkeit aus einem Flüssigkeitsreservoir herauszusaugen, bestimmt. Dies geschieht mittels einer saugflächenreduzierten Zylindereinheit. Im Gegensatz zur üblichen Methode zur Bestimmung der Absorption gegen Druck (Absorption Against Pressure) wird auch der Flüssigkeitstransport in xy- Richtung ( Permeabilität ) in der Gelschicht betrachtet. Als Zylindereinheit wird bei der Messung die in der EP 640 330, S. 14, beschriebene Meßapparatur benutzt.
Diese Apparatur wird dahingehend modifiziert, daß ein Teil des Siebbodens mit einer flüssigkeitsundurchlässigen Schicht abgedeckt wird. In der Mitte des Siebbodens bleibt, zentrisch angeordnet, eine kreisrunde Öffnung, durch die die Flüssigkeitsaufnahme erfolgt. Die verbleibende Saugfläche kann z. B. 9, 14, 17, 25, 34 oder 60 % der ursprünglichen Fläche von 28,27 cm$^2$ betragen.
Die prozentuale Saugfläche, bezogen auf die übliche AAP- Fläche von 28,27 cm$^2$ wird als Index Ax angegeben:

- -   AAP- A$_9$ ( Saugfläche 18 mm Ø)
- -   AAP- A$_{14}$ ( Saugfläche 23 mm Ø )
- -   AAP- A$_{17}$ ( Saugfläche 25 mm Ø)
- -   AAF- A$_{25}$ ( Saugfläche 30 mm Ø)
- -   AAP- A$_{34}$ ( Saugfläche 35 mm Ø )
- -   AAP- A$_{60}$ ( Saugfläche 46,5 mm Ø )

Es werden 0,900 ± 0,005 g des Superabsorbers eingewogen und möglichst gleichmäßig auf das Siebgewebe des Plastikzylinders (Ø = 6 cm, Höhe = 5 cm, Siebgewebe: 400 mesh = 37 µm) im Boden gestreut und mit einem definierten Gewicht belastet. Die Belastung beträgt wahlweise 20 g/cm$^2$ bzw. 50 g/cm$^2$.
Die Zylindereinheit (Plexiglaszylinder mit flächenreduziertem Siebgewebe, SAP, Abdeckplatte und Gewicht) wird gewogen und auf eine mit Filterpapier (Schleicher und Schüll, Schwarzband 589; Ø = 11 cm) abgedeckte, mit Flüssigkeit getränkte Filterplatte (Ø = 12 cm, Porosität = 0; z. B. Schott-Keramikfilter Duran) gestellt. Die Filterplatte liegt bis zu ihrer Oberkante in der Flüssigkeit. In der Regel wird 0,9 %ige NaCl-Lösung verwendet, andere Prüflösungen wie z. B. synthetischer Urin sind ebenfalls verwendbar. Überstehende Flüssigkeit ist zu vermeiden.
Man läßt das SAP eine definierte Zeit saugen. In der Regel beträgt die Standzeit 60 Minuten; optional sind andere Zeiträume möglich.Durch Rückwaage der Zylindereinheit kann die Absorption gegen Druck bei reduzierter Saugfläche (AAP-A$_x$) bestimmt werden. Es ist jeweils mindestens eine Doppelbestimmung durchzuführen. Zur Berechnung der prozentualen Kapazität wird auch der AAP- Wert mit der entsprechenden Belastung ermittelt.

Die Berechnung des flächenreduzierten AAP-$A_x$ Wertes erfolgt mit folgender Formel:

$$\{AAP - A_x\} = \frac{B - A}{E} \; [g/g]$$

$$Kapazit\ddot{a}t = \frac{\{AAP - A_x\} * 100}{AAP} \; [\%]$$

{AAP- AX} :     Absorption gegen Druck bei reduzierter Saugfläche [g/g]

A :     Gewicht der Zylindergruppe vor dem Saugen [g]

B :     Gewicht der Zylindergruppe nach dem Saugen [g]

E :     Einwaage des Superabsorbers [g]

**7. Stabilitätsprüfung der Oberflächenvernetzung (SDOV)**

Ein zylindrischer Hohlkörper aus Korund mit einem inneren Durchmesser und einer inneren Länge von je ca. 8 cm wird mit 20 g Superabsorberpulver und etwa 130 g zylindrischen Korund-Körpern, die einen Durchmesser bzw. eine Länge von je 1,27 cm haben, gefüllt und mit einer Geschwindigkeit von 150 U/min gedreht. Nach 10 Minuten beendet man den Stabilitätstest und bestimmt die Absorption unter Druck (AUL 63).

**8. Geschwindigkeit der Flüssigkeitsaufnahme (SG)**

Bei diesem Test wird die Zeit gemessen, in der 1 g Superabsorber 20g einer 0,9%igen Kochsalzlösung bei Raumtemperatur aufsaugt. Der Ablauf dieser Prüfung ist in der EP 443 627, Seite 12, "Free-Swell-Rate", geschildert.

**Beispiele**

**Vergleichsbeispiel 1**

a) 400 kg/h einer 33%igen Monomerlösung aus mit Natronlauge zu 70 Mol-% teilneutralisierter Acrylsäure, 3,5 Gew.-% (bezogen auf Acrylsäure) Methoxypolyethylenglykol(22EO)methacrylat, 0,35 Gew.-% (bezogen auf Acrylsäure) Trimethylolpropan-triacrylat und 0,4 Gew.-% Natriumcarbonat (bezogen auf Acrylsäure) werden kontinuierlich mit Stickstoff gespült und bei 4 - 5 °C mit folgenden Katalysatorlösungen vermischt: 100 ppm Wasserstoffperoxid, 150 ppm Natriumperoxidisulfat und 100 ppm Azoisobutyroamidindihydrochlorid. Zur kontinuierlichen Polymerisation auf einem Endlosband werden 15 ppm Ascorbinsäure zugegeben. Nach 40 Minuten Polymerisationszeit wird das entstandene Gel zerkleinert und auf einem Bandtrockner bei 160 °C Lufttemperatur getrocknet.

Nach dem Mahlen und Absieben auf 150 - 850 µm wird das Polymer zwischengelagert.

| Eigenschaften des Vorproduktes: | |
| --- | --- |
| Retention | 39,5 g/g |
| Lösliche Anteile nach 1 Std. | 9,3 % |
| Lösliche Anteile nach 16 Std. | 14,1 % |

b) Nachvernetzung des Vorproduktes:

Das nach Vergleich **1 a)** gewonnene pulverförmige Polymer wird bei einem Durchsatz von 80 kg/h in einem Paddelmischer (2000 U/min) kontinuierlich mit 1,5 % einer Lösung aus 1 TL Ethylencarbonat und 2 TL Wasser besprüht und in einem mit beheizten Mischelementen ausgerüsteten Paddeltrockner erwärmt.

| Dampftemperatur | 190 °C |
| --- | --- |
| Heizfläche | 1,6 m$^2$ |
| mittlere Verweilzeit | 20 min. |

Nach Kühlung des Produktes erfolgt eine Schutzsiebung bei 850 µm.
Eigenschaften des nachvernetzten Produktes:

| Retention | 33,5 g/g |
|---|---|
| AUL (21 g/cm$^2$) | 31 g/g |
| AUL (63 g/cm$^2$) | 18 g/g |
| Lösliche Anteile nach 1 Std. | 6,4 % |
| Lösliche Anteile nach 16 Std. | 11,0 % |
| SG | 33 s |
| AUL (63 g/cm$^2$)n. SDOV | 12 g/g |
| QD (20') | 496 g |

**Vergleichsbeispiel 2**

a) Wie in Vergleich 1 a) werden 400 kg/h einer 33%igen Monomerlösung aus mit Natronlauge zu 70 Mol-% teilneutralisierter Acrylsäure und 0,3 Gew.-% 15EO-Trimethylolpropantriacrylat kontinuierlich initiiert, polymerisiert und zu einem pulverförmigen Harz aufgearbeitet.

| Retention | 42 g/g |
|---|---|
| Lösliche Anteile nach 1 Std. | 12,0 % |
| Lösliche Anteile nach 16 Std. | 19,5 % |

b) Nachvernetzung
Das nach Vergleich **2 a)** gewonnene pulverförmige Polymer wird wie in Vergleich 1 b) behandelt. Die resultierenden Produkteigenschaften sind wie folgt:

| Retention | 31 g/g |
|---|---|
| AUL (21 g/cm$^2$) | 30 g/g |
| AUL (63 g/cm$^2$) | 17 g/g |
| Lösliche Anteile nach 1 Std. | 6,5 % |
| Lösliche Anteile nach 16 Std. | 20,5 % |
| SG | 62 s |
| AUL (63 g/cm$^2$) nach SDOV | 12 g/g |
| QD (20') | 528 g |

**Beispiel 1**

a) Wie in Vergleichsbeispiel 1 a) wird eine 70 Mol-% teilneutralisierte Acrylsäurelösung, die - bezogen auf Acrylsäure - 3,5 % Methoxypolyethylenglykol(22EO)methacrylat, 0,2 % Trimethylolpropan-triacrylat, 0,3 % Polyethlenglykol(10EO) monoallylether-acrylat enthält, polymerisiert und zu einem pulverförmigen Harz mit den folgenden Eigenschaften aufgearbeitet:

| Retention | 41 g/g |
|---|---|
| Lösliche Anteile nach 1 Std. | 6,1 % |
| Lösliche Anteile nach 16 Std. | 9,5 % |

b) Nachvernetzung
Das nach Beispiel 1 a) gewonnene pulverförmige Polymer wird wie in Vergleichsbeispiel 1 b) nachbehandelt und hat dann die folgenden Eigenschaften:

| Retention | 34 g/g |
|---|---|
| AUL (21 g/cm$^2$) | 34 g/g |
| AUL (63 g/cm$^2$) | 25,5 g/g |
| Lösliche Anteile nach 1 Std. | 4,8 % |
| Lösliche Anteile nach 16 Std. | 9,4 % |
| SG | 30 s |
| AUL (63 g/cm$^2$) nach SDOV | 19 g/g |
| QD (20') | 810 g |

**Beispiel 2**

a) Wie in Vergleichsbeispiel 1a) werden 400 kg/h einer zu 70 Mol-% teilneutralisierten Acrylsäurelösung, die - bezogen auf Acrylsäure - 3,5 % Methoxypolyethylenglykol(22EO)methacrylat, 0,2 % Trimethylolpropan-triacrylat und 0,4 % Polyethlenglykol(10EO)-monoallylether-acrylat enthält, polymerisiert, getrocknet, gemahlen und auf die Kornfraktion 150 - 850 μm abgesiebt.

| Retention | 38,5 g/g |
|---|---|
| Lösliche Anteile nach 1 Std. | 5,8 % |
| Lösliche Anteile nach 16 Std. | 7,6 % |

b) Nachvernetzung:
Das nach Beispiel 2 gewonnene Polymer wird wie in Vergleichsbeispiel 1 b) beschichtet und thermisch behandelt. Es hat die folgenden Kenndaten:

| Retention | 32 g/g |
|---|---|
| AUL (21 g/cm$^2$) | 33,5 g/g |
| AUL (63 g/cm$^2$) | 25,5 g/g |
| Lösliche Anteile nach 1 Std. | 4,8 % |
| Lösliche Anteile nach 16 Std. | 7,0 % |
| SG | 33 s |
| AUL (63 g/cm$^2$) nach SDOV | 20 g/g |
| QD (20') | 960 g |

**Beispiel 3 (W 75067)**

a) Wie in Vergleichsbeispiel 1 a) werden 400 kg/h einer zu 70 Mol-% teilneutralisierten Acrylsäurelösung, die - bezogen auf Acrylsäure - 3,5 % Methoxypolyethylenglykol(22EO)-methacrylat, 0,3 % 3-EO-Trimethylolpropan-triacrylat und 0,4 % Polyethlenglykol(10EO)-monoallylether-acrylat enthält, durch Zugabe der in Vergleichsbeispiel 1 a) genannten Initiatoren polymerisiert. Das erhaltene Gel wird zerkleinert, getrocknet, gemahlen und auf 150 - 850 μm abgesiebt. Es wurden an dem Vorprodukt die folgenden Eigenschaften bestimmt:

| Retention | 36 g/g |
|---|---|
| Lösliche Anteile nach 1 Std. | 5,0 % |
| Lösliche Anteile nach 16 Std. | 6,6 % |

b) Nachvernetzung:
Wie in Vergleichsbeispiel 1 b) werden 80 kg/h Polymer aus Beispiel 3 a) mit 1,5 % Ethylencarbonatlösung vermischt und anschließend im Paddeltrockner erwärmt. Das gewonnene Polymer zeigt folgende Kenndaten:

| Retention | 32 g/g |
|---|---|
| AUL (21 g/cm$^2$) | 34 g/g |
| AUL (63 g/cm$^2$) | 24 g/g |
| Lösliche Anteile nach 1 Std. | 2,7 % |
| Lösliche Anteile nach 16 Std. | 6,8 % |
| SG | 28 s |
| AUL (63 g/cm$^2$) nach SDOV | 18 g/g |

In den Beispielen 4 - 20 und Vergleichsbeispielen 3 - 8 wird nach der folgenden Richtrezeptur polymerisiert und nachvernetzt:

In einem zylindrischen Kunststoffgefäß wird ein Polymerisationsansatz von insgesamt 1000g zubereitet. Dazu werden 280 g Acrylsäure sowie die verwendeten Vernetzer, Comonomere und weitere Komponenten in vollentsalztem Wasser angesetzt. Unter Rühren und Kühlen wird mit 50%-iger Natronlauge bis zu einem Neutralisationsgrad von 70 % teilneutralisiert. Die Lösung wird auf 7-8°C abgekühlt und mit Stickstoff solange durchperlt, bis der Sauerstoffgehalt in der Monomerenlösung auf einen Wert von unter 0,2 ppm abgesunken ist. Anschließend gibt 100 ppm Azo-bis ( 2-amidinopropan)dihydrochlorid gelöst in 10 g VE-Wasser, 300 ppm Natriumpersulfat, gelöst in 6 g VE-Wasser, 70 ppm Wasserstoffperoxid (35%-ig) gelöst in 1 g VE-Wasser hinzu. Danach wird die Polymerisation durch Zugabe von 9 ppm Ascorbinsäure gelöst in 2g Wasser gestartet, worauf eine deutliche Temperaturerhöhung eintritt. Nach Beendigung der Polymerisation wird der gelartige Polymerblock zerkleinert, gewölft und getrocknet. Das Polymer wird anschließend gemahlen und auf die Kornfraktion 150 - 800 μ abgesiebt.

Nachvernetzung:

100 g des abgesiebten Polymeren wird unter kräftigem Durchmischen mit einer Lösung aus 0,5 g Ethylencarbonat, 2 g VE-Wasser und 4 g Aceton vermischt und anschließend für 25 Minuten in einem Ofen auf eine Temperatur von 180°C erhitzt.

Die Zusammensetzung der Superabsober bezüglich der Vernetzer, Comonomere und weiterer Komponenten sowie die Produkteigenschaften sind in den Tabellen 1 bis 3 aufgeführt.

Man erkennt aus der Tabelle, daß die erfindungsgemäßen Beispiele Polymerisate mit einer Kombination guter Eigenschaften liefern:

| Retention | >30 g/g |
|---|---|
| Absorption unter Druck (49 g/cm$^2$) | >20 g/g |
| Absorption unter Druck (63 g/cm2) nach SDOV | >18 g/g |
| Geschwindigkeit der Flüssigkeitsaufnahme | <40 s |
| Lösliche Anteile (16 h) | <10 % |
| Quelldruck (20 Min) | >800 g |

Bei den Vergleichsbeispielen zeigt es sich, daß zwar durch Variation der Vernetzermenge einzelne Meßwerte, aber nicht die gesamte Kombination der erfindungsgemäßen guten Eigenschaften zu erreichen ist.

**Beispiel 21 - 24**

Entsprechend dem Beispiel 1 wurden weitere Versuche mit folgenden Vernetzer/Monomerkombinationen durchgeführt, wobei die Mengenangaben Gew.% auf Acrylsäure bedeuten:

| Vernetzer/ Comonomer | Beispiel 21 (Code W 75066) | Beispiel 22 (Code W 75069) | Beispiel 23*(Code W 76164) | Beispiel 24 (Code W 76165) |
|---|---|---|---|---|
| TMPTA-15EO | 0,4 | | | |
| TMPTA-3EO | | | | 0.3 |
| PE-5EO-TA | | | 0,14 | |

* : Beispiel 24 wurde ohne Zusatz von Natriumcarbonat durchgeführt

(fortgesetzt)

| Vernetzer/ Comonomer | Beispiel 21 (Code W 75066) | Beispiel 22 (Code W 75069) | Beispiel 23*(Code W 76164) | Beispiel 24 (Code W 76165) |
|---|---|---|---|---|
| AA-10EO-A | 0,2 | 0,2 | 0,4 | 0,4 |
| TMPTA | | 0,2 | | |
| MPEG1000 MA | 3,5 | 3,5 | 2,9 | 1,67 |
| Produkteigenschaft (nachvernetzt) | | | | |
| Retention [g/g] | 32,5 | 33,5 | 34 | 29,5 |
| AUL 63 [g/g] | 25,5 | 25 | 23,5 | 25 |
| AUL 63 n. SDOV [g/g] | 20 | 20 | 18 | 22 |
| AAP 50 $A_{34}$ [g/g] | | 16,5 | 17 | 23,5 |
| AAP 50 $A_{34}$ [%] | | 63 | 65 | 92 |
| LA 1 h [%] | 3,0 | 3,5 | 2,2 | 2,5 |
| LA 16 h [%] | 8,2 | 9,3 | 6,4 | 5,5 |

* : Beispiel 24 wurde ohne Zusatz von Natriumcarbonat durchgeführt

**Beispiel 25 -30**

Gemäß der allgemeinen Rezeptur aus den Beispielen 4 - 20 wurden weitere erfindungsgemäße superabsorbierende Polymere unter Mitverwendung von Triallylamin als Vernetzerkomponente III hergestellt. Im Beispiel 27 wurden nach der Neutralisation der Acrylsäure 0,4 g Natriumcarbonat zugefügt. Die Versuchsergebnisse sind in Tabelle 4 dargestellt.

**Beispiel 31**

Gemäß der allgemeinen Rezeptur von Beispiel 1 wurde ein Versuch mit einer Vernetzer/Monomerkombination aus 2,5 Gew. % Methoxypolyethylenglykol(22EO)-methacrylat, 0,35 Gew. % Polyethylenglykol(10EO)-monoallylether-acrylat und 0,12 Gew. % Triallylamin durchgeführt. Das Vorprodukt hatte eine Retention von 38 g/g, das nachvernetzte Produkt hatte eine Retention von 32,5 g/g, einen AUL (63 g/cm2) von 26,5 g/g einen AUL nach SDOV von 20 g/g, einen Quelldruck von 1340 g (n. 20') bzw. 1080 g (n. 2 h) und 6,4 % lösliche Anteile (nach 16 h).

**Vergleichsbeispiel 9 und Beispiele 32 - 34**

Anhand dieser Beispiele wird gezeigt, wie sich durch Verwendung von Methoxypolyethylenglycol-Methacrylat als Comonomer die Permeabilität der gequollenen Gelschicht verbessert. Ergebnisse sind in Tabelle 5 zusammengefaßt.

**Beispiel 32**

Gemäß der allgemeinen Rezeptur von Beispiel 1 wurde ein Versuch mit einer Vernetzer/Monomerkombination aus 1 Gew. % Methoxypolyethylenglykol(17EO)-methacrylat, 0,3 Gew. % Polyethylenglykol(10EO)-monoallylether-acrylat und 0,1 Gew. % 3-EO-Trimethylolpropantriacrylat durchgeführt. Das Vorprodukt hatte eine Retention von 37,5 g/g, das nachvernetzte Produkt hatte eine Retention von 32,5 g/g, einen AUL (63 g/cm$^2$) von 23,5 g/g und 8,5 % lösliche Anteile (nach 16 h).

**Beispiel 33**

Gemäß der allgemeinen Rezeptur von Beispiel 1 wurde ein Versuch mit einer Vernetzer/Monomerkombination aus 2 Gew. % Methoxypolyethylenglykol(17EO)-methacrylat, 0,3 Gew. % Polyethylenglykol(10EO)-monoallylether-acrylat und 0,1 Gew. % 3-EO-Trimethylolpropantriacrylat durchgeführt. Das Vorprodukt hatte eine Retention von 37,5 g/g, das nachvernetzte Produkt hatte eine Retention von 31,5 g/g, einen AUL (63 g/cm$^2$) von 24,5 g/g und 8,5 % lösliche Anteile (nach 16 h).

**Beispiel 34**

Gemäß der allgemeinen Rezeptur von Beispiel 1 wurde ein Versuch mit einer Vernetzer/Monomerkombination aus 3 Gew. % Methoxypolyethylenglykol(17EO)-methacrylat, 0,3 Gew. % Polyethylenglykol(10EO)-monoallylether-acrylat und 0,1 Gew. % 3-EO-Trimethylolpropantriacrylat durchgeführt. Das Vorprodukt hatte eine Retention von 39 g/g, das nachvernetzte Produkt hatte eine Retention von 32 g/g, einen AUL (63 g/cm$^2$) von 23,5 g/g und 6,5 % lösliche Anteile (nach 16 h).

**Vergleichsbeispiel 9**

Gemäß der allgemeinen Rezeptur von Beispiel 1 wurde ein Versuch mit einer Vernetzer/Monomerkombination ohne Methoxypolyethylenglykol(17EO)-methacrylat, bestehend aus 0,3 Gew. % Polyethylenglykol(10EO)-monoallylether-acrylat und 0,1 Gew. % 3-EO-Trimethylolpropan-triacrylat durchgeführt. Das Vorprodukt hatte eine Retention von 33 g/g, das nachvernetzte Produkt hatte eine Retention von 32 g/g, einen AUL (63 g/cm$^2$) von 19,5 g/g und 7,6 % lösliche Anteile (nach 16 h).

**Vergleichsbeispiele 10 - 12**

An kommerziell erhältlichen Superabsorbern wird der AAP50-A$_{34}$ bestimmt. Die Meßwerte belegen, daß die Produkte die erfindungsgemäß geforderten Grenzwerte nicht erreichen (Tabelle 6).

**Tabelle 1**

| Beisp. | Vernetzer III TMPTA-3EO [Gew.%] | Vernetzer I AA-10EO-MA / AA-10EO-A [Gew%] | Monomer II MPEG1000 MA [Gew%] | Vorprodukt Retention [g/g] | Nachvernetztes Produkt | | | | | | | |
|---|---|---|---|---|---|---|---|---|---|---|---|---|
| | | | | | Retention [g/g] | AUL 63 [g/g] | AUL 63 n.SDOV | AAP 50 A$_{34}$ [g/g] | AAP 50 A$_{34}$ [%] | RM Acryls. [ppm] | L.A. 1 h [%] | L.A. 16 h [%] |
| 4 | 0,2 | 0 | 3 | 38,4 | 30,2 | 25,5 | 20,5 | | | 960 | | 8,6 |
| 5 | 0,1 | 0,2 A | 3 | 40,6 | 31,2 | 26,5 | 23 | | | 510 | 3,8 | 9,2 |
| 6 | 0,15 | 0,25 A | 3 | 37,1 | 30,5 | 25 | 24 | 22,5 | 94 | 640 | 3,1 | 7,1 |
| Vergl. 3 | 0,15 | 0,25 A | 0 | 32,3 | 28,6 | 25,5 | 22 | | | 1610 | | 7,7 |
| 7 | 0,15 | 0,25 MA | 3 | 38,1 | 30,6 | 25,5 | 24 | | | 940 | | 8,8 |
| Vergl.4 | 0,15 | 0,25 | 3 (MAC 13/20) | 40 | 34 | 22,5 | 15 | | | 800 | | 13 |
| Vergl. 5 | 0,15 | 0,25 A | 1,5 (MAC 13/20) | 38,8 | 31,8 | 25,5 | 17,5 | | | 1050 | | 12 |

TMPTA-3EO: Triacrylat eines mit 3Mol EO ethoxylierten Trimethylopropans

AA-10EO-MA: Methacrylatester eines mit 10 Mol EO ethoxylierten Allylalkohols

AA-10EO-A: Acrylatester eines mit 10 Mol EO ethoxylierten Allylalkohols

MPEG 1000 MA: Methoxypolyethylenglykol( 22 Mol EO)methacrylat

MAC13/20: C13-Polyethylenglkol(20 Mol EO)methacrylat

EP 0 862 590 B1

## Tabelle 2

| Beispiel | Vernetzer III PE-5EO-TA [Gew. %] | Vernetzer I AA-10EO-MA AA-10EO-A [Gew. %] | Monomer II MPEG1000 MA [Gew. %] | Vorprodukt Retention [g/g] | Nachvernetztes Produkt Retention [g/g] | AUL 63 [g/g] | AUL 63 n. SDOV [g/g] | RM Acryls. [ppm] | LA 16 h [%] |
|---|---|---|---|---|---|---|---|---|---|
| 8 | 0,15 | 0,25 A | 3 | 38,7 | 31,5 | 24,5 | 19 | 605 | 9 |
| 9 | 0,1 | 0,35 A | 3 | 36,2 | 32 | 23,5 | 20,5 | 660 | 8 |
| 10 | 0,15 | 0,25 MA | 3 | 37,2 | 31 | 24 | 19,5 | | 8,5 |
| 11 | 0,15 | 0,3 MA | 3 | 36,8 | 31 | 25 | 20 | | 7,7 |
| 12* | 0,1 | 0,35 A | 2 | 37,4 | 30,2 | 24 | 19 | 800 | 8,3 |
| 13* | 0,1 | 0,35 MA | 2 | 36,2 | 30,9 | 24 | 18,5 | 800 | 8,4 |
| Vergl. 6 | 0,1 | 0,35 A | 0 | 32,7 | 30,5 | 21 | 16,5 | 650 | 6,5 |
| 14 | 0,1 | 0,3 A + 0,05 MAPEG-MA | 3 | 38,5 | 31 | 24,5 | 20,5 | 740 | 8,9 |
| Vergl. 7 | 0,1 | 0,3 A + 0,05 MAPEG-MA | 0 | 31,7 | 30 | 22,5 | 16,5 | 970 | 6,9 |

EP 0 862 590 B1

| 15 | 0,1 | 0,25 MAPEG-MA | 3 | 35 | 32,2 | 24,5 | 18 | 1200 | 9 |

PE-5EO-TA: Pentaerythrit-pentaethoxylat-tetraacrylat

PVA: Polyvinylalkohol

MAPEG-MA: Methacrylpolyethylenglykol(15EO)-methacrylat

*: Beispiel 12 und 13 wurden mit 0,5 % Polyvinylalkohol (Mowiol 5/88) hergestellt.

## Tabelle 3

| Beispiel | Vernetzer III TMPTA-15EO | Monomer II MPEG 1000 MA [Gew%] | Vorprodukt Retention [g/g] | Nachvernetztes Produkt | | | | | | | | | | | |
|---|---|---|---|---|---|---|---|---|---|---|---|---|---|---|---|
| | | | | Retention [g/g] | AUL 63 [g/g] | AUL 63 n.SDOV [g/g] | AAP 50 A₃₄ [g/g] | AAP 50 A₃₄ [%] | Quelldruck 20' | 2 h | 4 h | 16 h | RM Acryls [ppm] | LA 1 h [%] | LA 16 h [%] |
| Vergl. 8 | 0,25 | 0 | 36,6 | 30,5 | 26,5 | 16,5 | | | 1225 | 975 | 850 | 590 | 1100 | 4 | 10 |
| 16 | 0,25 | 1,5 | 38,2 | 31 | 26,5 | 21 | 23,5 | 89 | 1125 | 925 | 812 | 575 | 960 | 4,5 | 9 |
| 17 | 0,25 | 3 | 40,1 | 31,2 | 26 | 22,5 | | | 1095 | 865 | | | 770 | 4,5 | 9 |
| 18 | 0,25 | 5 | 40,3 | 31,2 | 25,5 | 24 | 21,5 | 98 | 1095 | 713 | | | 590 | 5 | 9,5 |
| 19 | 0,25 | 3 (MPEG 350 MA) | 38 | 31,2 | 25 | 22 | | | | | | | 755 | 3,6 | 8,6 |
| 20 | 0,25 | 3 (MPEG 2000 MA) | 37,6 | 32,2 | 25 | 22 | | | | | | | 805 | 4,5 | 9,4 |

TMPTA-15EO: Triacrylat eines mit 15 Mol EO ethoxylierten Trimethylolpropans

MPEG 350 MA: Methacrylatester eines Methoxypolyethylenglykols mit 8 Mol EO

MPEG 2000 MA: Methacrylatester eines Methoxypolyethylenglykols mit 45 Mol EO

EP 0 862 590 B1

## Tabelle 4

| Beispiel | Vernetzer I u.III | Monomer II | Vorprodukt | Nachvernetztes Produkt | | | | | | | |
|---|---|---|---|---|---|---|---|---|---|---|---|
| | TAA / AA-10EO-A/ [Gew%] | MPEG 1000 MA | Retention [g/g] | Retention [g/g] | AUL 63 [g/g] | AUL 63 n.SDOV [g/g] | Quelldruck [g] 20'  2 h  4 h  16 h | | RM Acryls. [ppm] | LA 1 h [%] | 16 h [%] |
| 25 | 0,1/0,2/0 | 2 | 38,5 | 32 | 27 | 24 | 1300 1060 | | 400 | | 7,2 |
| 26 | 0.1/0,3/0 | 2 | 38 | 31,5 | 26,5 | 23,5 | | | 430 | | 5,8 |
| 27 | 0,1/0,3/0 | 2 | 37,5 | 32 | 27 | 23 | | | | | |
| 28 | 0,1/0,4/0 | 2 | 36,5 | 31 | 26,5 | 22 | | | 450 | | 5,9 |
| 29 | 0,1/0,1/ 0,1(PEG600DA) | 2 | 38,5 | 33 | 27 | 21 | | | 520 | | 6,0 |
| 30 | 0,1/0,15/ 0,05(TMPTA) | 2 | 36,5 | 31,5 | 26 | | | | 800 | | 5,5 |

TMPTA: Trimethylolpropantriacrylat

TAA: Triallylamin

PEG600DA: Diacrylatester eines Polyethylenglykols des Molgew. 600

AA-10EO-A: Acrylatester eines mit 10 Mol EO ethoxylierten Allylalkohols

MPEG 1000 MA: Methacrylatester eines Methoxypolyethylenglykols mit 22 Mol EO

EP 0 862 590 B1

## Tabelle 5

| Beispiel | Vernetzer III TMPTA-3EO [Gew.%] | Vernetzer I AA-10EO-A [Gew%] | Monomer II MPEG750 MA [Gew%] | Vorprodukt Retention [g/g] | nachvernetztes Produkt | | | | | |
|---|---|---|---|---|---|---|---|---|---|---|
| | | | | | Retention [g/g] | AUL 21 [g/g] | AUL 63 [g/g] | AAP 50 $A_{34}$ [g/g] | AAP 50 $A_{34}$ [%] | L.A. 16 h [%] |
| Vergl. 9 | 0,1 | 0,3 | 0 | 33 | 32 | 31,5 | 19,5 | 10,5 | 48 | 7,6 |
| 32 | 0,1 | 0,3 | 1 | 37,5 | 32,5 | 32 | 23,5 | 19,5 | 76 | 8,5 |
| 33 | 0,1 | 0,3 | 2 | 37,5 | 31,5 | 32 | 24,5 | 20 | 80 | 8,5 |
| 34 | 0,1 | 0,3 | 3 | 39 | 32 | 32 | 23,5 | 21 | 84 | 6,4 |

TMPTA-3EO: Triacrylat eines mit 3 Mol EO ethoxylierten

Trimethylopropans

AA-10EO-A: Acrylatester eines mit 10 Mol EO ethoxylierten Allylalkohols

MPEG 750 MA: Methoxypolyethylenglykol (17 Mol EO)

methacrylat

EP 0 862 590 B1

EP 0 862 590 B1

Tabelle 6

| Beispiel | Handels-Produkt | AAP 50 - $A_{34}$ [g/g] | AAP 50 - $A_{34}$ [%] |
|---|---|---|---|
| Vergleich 10 | Salsorb CL 20 | 8,5 | 63 |
| Vergleich 11 | ASAP 2000 | 14 | 74 |
| Vergleich 12 | Sanwet IM 7000 | 6,5 | 43 |

**Patentansprüche**

1. Wäßrige Flüssigkeiten absorbierendes vernetztes Polymerisat, aufgebaut aus teilneutralisierten, monoethylenisch ungesättigten Säuregruppen tragenden Monomeren, gegebenenfalls weiteren, damit copolymerisierbaren Monomeren sowie gegebenenfalls als Pfropfgrundlage geeigneten Polymeren, dadurch gekennzeichnet, daß es unter Verwendung einer Vernetzer/Monomer-Kombination aus

$$I. \qquad CH_2=CR^6-CO(OCHR^3-CHR^3)_z O-CH_2-CR^6=CH_2$$

$$II. \qquad CH_2=CR^6-R^5-(OCHR^3-CHR^3)_v OR^4$$

$$III. \qquad R^1-[O(CHR^3-CHR^3O)_u-CO-R^2]_x,$$

und/oder Di- bzw. Triallylamin und/oder Bisacrylamide
mit

$R^1$ : mehrwertiges C2-10-Alkyl,
$R^2$: linear oder verzweigt C2-10-Alkenyl,
$R^3$: H, $CH_3$, $C_2H_5$,
$R^4$: H, linear oder verzweigt C1-10-Alkyl,
$R^5$: CO, $CH_2$,
$R^6$: H, $CH_3$,
x: 2-6,
u: 0-15,
v: 1-45,
z: 3-20

herstellbar ist.

2. Polymerisat nach Anspruch 1, dadurch gekennzeichnet, daß die Komponenten

I) mit 0 - 1,0 Gew. %, vorzugsweise mit 0,05 - 0,6 Gew. %,
II) mit 0,1 - 10 Gew. %, vorzugsweise mit 0,5 - 5 Gew. % und
III) mit 0,01 - 1,0 Gew. %, vorzugsweise mit 0,05 bis 0,6 Gew. %

bezogen auf die Monomeren eingesetzt werden.

3. Polymerisat nach Anspruch 1, dadurch gekennzeichnet, daß die Vernetzer

I) mit 0,1- 0,4 Gew. %,
II) mit 1,0 - 3,5 Gew. % und
III) mit 0,05 - 0,3 Gew. %

bezogen auf die Monomeren eingesetzt werden.

21

4. Polymerisat nach einem der Ansprüche 1 bis 3, dadurch gekennzeichnet, daß die Vernetzer nach I aus der Gruppe der Allylpolyethylenglykol-(meth)acrylsäureester, daß die Monomere nach II aus der Gruppe der Methylpolyethylenglykol-(meth)acrylate und daß die Vernetzer nach III aus der Gruppe der Trimethylolpropan-oxethylat-(meth)acrylsäureester, der Glycerin-oxethylat(meth)acrylsäureester, der Pentaerythrit-oxethylat-(meth)acrylsäureester, der Polyethylenglykol-$\alpha,\omega$ -di(meth)acrylsäureester und Di- bzw. Triallylamin, N,N-Methylenbisacrylamid und Bisacrylamidoessigsäure ausgewählt sind.

5. Polymerisat nach einem der Ansprüche 1 bis 4, dadurch gekennzeichnet, daß die ungesättigten Säuregruppen tragenden Monomeren aus der Gruppe Acrylsäure, Methacrylsäure, Vinylessigsäure, Vinylsulfonsäure, Methallylsulfonsäure und 2-Acrylamido-2-methylpropansulfonsäure ausgewählt sind.

6. Polymerisat nach einem der Ansprüche 1 bis 5, dadurch gekennzeichnet, daß es 0 bis 40 Gew. % weitere Comonomere aus der Gruppe (Meth)acrylamid, (Meth)acrylnitril, Vinylpyrrolidon, Hydroxyethylacrylat und Vinylacetamid einpolymerisiert enthält.

7. Polymerisat nach einem der Ansprüche 1 bis 6, dadurch gekennzeichnet, daß es bis zu 30 Gew.% wasserlöslicher Polymere als Pfropfgrundlage, vorzugsweise Polysaccharide und/oder Polyvinylalkohol enthält.

8. Polymerisat nach einem der Ansprüche 1 bis 7, dadurch gekennzeichnet, daß es an der Oberfläche mit einem Nachvernetzer vernetzt wurde und diese Nachvernetzung gegebenfalls mehrfach wiederholt wurde.

9. Polymerisat nach Ansprch 8, dadurch gekennzeichnet, daß es an der Oberfläche mit einem Nachvernetzer aus der Gruppe der Polyole, Polyepoxide, Polyamine oder Alkylencarbonate vernetzt wurde.

10. Polymerisat nach einem der Ansprüche 8 und 9, dadurch gekennzeichnet, daß es eine Retention von mindestens 30 g/g, eine Flüssigkeitaufnahme unter Druck (63 g/cm$^2$) von mindestens 20 g/g und eine Flüssigkeitsaufnahme unter Druck (63 g/cm$^2$) nach SDOV von mindestens 18 g/g hat sowie lösliche Anteile nach 1 Stunde von höchstens 6,5 % und nach 16 Stunden von höchstens 10 % aufweist, sowie eine Absorption gegen einen Druck von 50 g/cm$^2$ bei einer auf 34 % reduzierten Saugfläche (AAP-A$_{34}$) von mindestens 15 g/g und mindestens 50% des Ausgangswertes der Absorption gegen Druck ohne Flächenreduzierung besitzt.

11. Polymerisat nach einem der Ansprüche 8, 9 und 10, dadurch gekennzeichnet, daß es eine Flüssigkeitsaufnahme unter Druck (63 g/cm$^2$) nach SDOV von mindestens 20 g/g, vorzugsweise von mindestens 22 g/g aufweist und einen AAP-A$_{34}$ von mindestens 20 g/g besitzt.

12. Polymerisat nach einem der Ansprüche 8, 9 und 10, dadurch gekennzeichnet, daß es einen Quelldruck (20 Min.) von mindestens 600 g, bevorzugt von mindestens 800 g und besonders bevorzugt von größer 900 g aufweist.

13. Polymerisat nach einem der Ansprüche 8, 9 und 10, dadurch gekennzeichnet, daß die löslichen Anteile nach 16 Stunden höchstens 8 % betragen.

14. Polymerisat nach einem der Ansprüche 8, 9 und 10, dadurch gekennzeichnet, daß es eine Flüssigkeitsaufnahme unter Druck (63 g/cm$^2$) von größer 23 g/g und besonders bevorzugt von größer 25 g/g aufweist.

15. Polymerisat nach einem der Ansprüche 8, 9 und 10, dadurch gekennzeichnet, daß es eine Geschwindigkeit der Flüssigkeitsaufnahme von kleiner 40 sec, bevorzugt kleiner 35 sec und besonders bevorzugt von kleiner 30 sec aufweist.

16. Verfahren zur Herstellung eines wäßrige Flüssigkeiten absorbierenden, vernetzten Polymerisates nach einem der Ansprüche 1 bis 7, dadurch gekennzeichnet, daß eine wäßrige Lösung aus ungesättigten, Säuregruppen tragenden, teilneutralisierten Monomeren und einer Vernetzermischung aus

I.     $CH_2=CR^6\text{-}CO(OCHR^3\text{-}CHR^3)_z O\text{-}CH_2\text{-}CR^6=CH_2$

II.     $CH_2=CR^6\text{-}R^5\text{-}(OCHR^3\text{-}CHR^3)_v OR^4$

III. $R^1$-$[O(CHR^3$-$CHR^3O)_u$-$CO$-$R^2]_x$,

und/oder Di- bzw. Triallylamin und/oder Bisacrylamide

mit

R$^1$ : mehrwertiges C2-10-Alkyl,
R$^2$: linear oder verzweigt C2-10-Alkenyl,
R$^3$: H, CH$_3$, C$_2$H$_5$,
R$^4$: H, linear oder verzweigt C1-10-Alkyl,
R$^5$: CO, CH$_2$,
R$^6$: H, CH$_3$,
x: 2-6,
u: 0-15,
v: 1-45,
z: 3-20

unter Zusatz von Radikalbildnern nach dem Verfahren einer Lösungs- oder Suspensionspolymerisation zu einem Hydrogel polymerisiert, zerkleinert, getrocknet, gemahlen und gesiebt wird.

17. Verfahren nach Anspruch 16, dadurch gekennzeichnet, daß die Polymerisate mit einem Oberflächenvernetzer behandelt werden und Oberflächenvernetzung bei erhöhter Temperatur durchgeführt wird.

18. Verfahren nach Anspruch 17, dadurch gekannzeichnet, daß die Oberflächenbehandlung und Vernetzung mehrmals durchgeführt wird.

19. Verwendung der Polymerisate nach einem der Ansprüche 1 bis 15 als Absorptionsmittel für Wasser und wäßrige Flüssigkeiten.

20. Verwendung der Polymerisate nach einem der Ansprüche 1 bis 13 in Konstruktionen zur Aufnahme von Körperflüssigkeiten.

21. Verwendung der Polymerisate nach einem der Ansprüche 1 bis 15 als Wasser oder wäßrige Flüssigkeiten absorbierende Komponente in strom -oder lichtleitenden Kabeln, als Komponente in Verpackungsmaterialien, als Bodenverbesserungsmittel und bei der Pflanzenaufzucht.

22. Verwendung der Polymerisate nach einem der Ansprüche 1 bis 15 in Wasser oder wäßrige Flüssigkeiten absorbierenden, geschäumten oder nicht geschäumten Flächengebilden.

23. Verwendung der Polymerisate nach einem der Ansprüche 1 bis 15 als Trägersubstanz für Düngemittel oder andere Wirkstoffe, die wieder über einen längeren Zeitraum verzögert an die Umgebung abgegeben werden.

## Claims

1. A crosslinked polymer product which absorbs aqueous liquids and is constituted of partially neutralized monomers bearing monoethylenically unsaturated acid groups, optionally other monomers copolymerizable therewith, and optionally polymers suitable as a basis for grafting, characterized in that the polymer product can be produced using a crosslinker/monomer combination of

I. $CH_2$=$CR^6$-$CO(OCHR^3$-$CHR^3)_z O$-$CH_2$-$CR^6$=$CH_2$

II. $CH_2$=$CR^6$-$R^5$-$(OCHR^3$-$CHR^3)_v OR^4$

III. $R^1$-$[O(CHR^3$-$CHR^3O)_u$-$CO$-$R^2]_x$,

and/or di- or tri-allylamine and/or bisacrylamides
wherein

$R^1$ : multivalent $C_{2-10}$ alkyl,
$R^2$: linear or branched $C_{2-10}$ alkenyl,
$R^3$: H, $CH_3$, $C_2H_5$,
$R^4$: H, linear or branched $C_{1-10}$ alkyl,
$R^5$: CO, $CH_2$,
$R^6$: H, $CH_3$,
x: 2-6,
u: 0-15,
v: 1-45,
z: 3-20.

2. The polymer product of claim 1, characterized in that the components are employed as follows:

I. at 0 - 1.0 wt.-%, preferably 0.05 - 0.6 wt.-%;
II. at 0.1 - 10 wt.-%, preferably 0.5 - 5 wt.-%; and
III. at 0.01 - 1.0 wt.-%, preferably 0.05 - 0.6 wt.-%;

relative to the monomers.

3. The polymer product of claim 1, characterized in that the crosslinkers are employed as follows:

I. at 0.1 - 0.4 wt.-%;
II. at 1.0 - 3.5 wt.-%; and
III. at 0.05 - 0.3 wt.-%;

relative to the monomers.

4. The polymer product according to any of claims 1 through 3, characterized in that the crosslinkers according to I. are selected from the group of allylpolyethylene glycol (meth)acrylates, the monomers according to II from the group of methylpolyethylene glycol (meth)acrylates, and the crosslinkers according to III from the group of trimethylolpropane oxethylate (meth)acrylates, glycerol oxethylate (meth)acrylates, pentaerythritol oxethylate (meth)acrylates, polyethyleneglycol α,ω-di(meth)acrylates, and di- or triallylamine, N,N-methylenebisacrylamide, and bisacrylamidoacetic acid.

5. The polymer product according to any of claims 1 through 4, characterized in that the monomers bearing unsaturated acid groups are selected from the group of acrylic acid, methacrylic acid, vinylacetic acid, vinylsulfonic acid, methallylsulfonic acid, and 2-acrylamido-2-methylpropanesulfonic acid.

6. The polymer product according to any of claims 1 through 5, characterized in that it contains polymerized therein from 0 to 40 wt.-% of other comonomers from the group of (meth)acrylamide, (meth)acrylonitrile, vinylpyrrolidone, hydroxyethyl acrylate, and vinylacetamide.

7. The polymer product according to any of claims 1 through 6, characterized in that it contains up to 30 wt.-% of water-soluble polymers as a basis for grafting, preferably polysaccharides and/or polyvinyl alcohol.

8. The polymer product according to any of claims 1 through 7, characterized in that it has been crosslinked at its surface with a secondary crosslinker, and this secondary crosslinking optionally has been repeated for several times.

9. The polymer product according to claim 8, characterized in that it has been crosslinked at its surface using a secondary crosslinker from the group of polyols, polyepoxides, polyamines, or alkylene carbonates.

10. The polymer product according to any of claims 8 and 9, characterized by a retention of at least 30 g/g, a liquid absorption under pressure (63 g/cm$^2$) of at least 20 g/g and a liquid absorption under pressure (63 g/cm$^2$) according to the stability test of surface crosslinking (STSC) of at least 18 g/g, solubles after 1 hour of 6.5% at maximum and after 16 hours 10% at maximum, an absorption against a pressure of 50 g/cm$^2$ on an absorbing area reduced to 34% (AAP-A$_{34}$) of at least 15 g/g, and at least 50% of the initial value of the absorption against pressure with no reduction of the area.

11. The polymer product according to any of claims 8, 9 and 10, characterized by a liquid absorption under pressure (63 g/cm$^2$) according to STSC of at least 20 g/g, preferably at least 22 g/g, and an of AAP-A$_{34}$ of at least 20 g/g.

12. The polymer product according to any of claims 8, 9 and 10, characterized by a swelling pressure (20 minutes) of at least 600 g, preferably at least 800 g, and more preferably above 900 g.

13. The polymer product according to any of claims 8, 9 and 10, characterized in that the solubles are 8% at maximum after 16 hours.

14. The polymer product according to any of claims 8, 9 and 10, characterized by a liquid absorption under pressure (63 g/cm$^2$) of more than 23 g/g, with more than 25 g/g being particularly preferred.

15. The polymer product according to any of claims 8, 9 and 10, characterized by a liquid absorption rate of below 40 s, preferably below 35 s, with less than 30 s being particularly preferred.

16. A process for producing an aqueous liquid-absorbing, crosslinked polymer product according to any of claims 1 through 7, characterized in that an aqueous solution of partially neutralized monomers bearing unsaturated acid groups and a mixture of monomers, comprising

$$\text{I.} \qquad CH_2=CR^6\text{-}CO(OCHR^3\text{-}CHR^3)_z O\text{-}CH_2\text{-}CR^6=CH_2$$

$$\text{II.} \qquad CH_2=CR^6\text{-}R^5\text{-}(OCHR^3\text{-}CHR^3)_v OR^4$$

$$\text{III.} \qquad R^1\text{-}[O(CHR^3\text{-}CHR^3O)_u\text{-}CO\text{-}R^2]_x,$$

and/or di- or triallylamine and/or bisacrylamides
wherein

R$^1$ : multivalent C$_{2\text{-}10}$ alkyl,
R$^2$: linear or branched C$_{2\text{-}10}$ alkenyl,
R$^3$: H, CH$_3$, C$_2$H$_5$,
R$^4$: H, linear or branched C$_{1\text{-}10}$ alkyl,
R$^5$: CO, CH$_2$,
R$^6$: H, CH$_3$,
x: 2-6,
u: 0-15,
v: 1-45,
z: 3-20

is polymerized with addition of free radical formers according to the procedure of a solution or suspension polymerization to form a hydrogel which is crushed, dried, milled, and screened.

17. The process according to claim 16, characterized in that the polymer products are treated with a surface crosslinker, and surface crosslinking is performed at elevated temperature.

18. The process according to claim 17, characterized in that surface treatment and crosslinking are performed several times.

19. Use of the polymer products according to any of claims 1 through 15 as an absorbent for water and aqueous liquids.

20. Use of the polymer products according to any of claims 1 through 13 in constructions for absorbing body fluids.

**21.** Use of the polymer products according to any of claims 1 through 15 as a component absorbing water or aqueous liquids used in electroconductive or light-conducting cables, as a component in packaging materials, as soil improver and in plant breeding,

**22.** Use of the polymer products according to any of claims 1 through 15 in foamed or non-foamed sheet materials absorbing water or aqueous liquids.

**23.** Use of the polymer products according to any of claims 1 through 15 as a supporting material for fertilizers or other active ingredients which are released into the environment in a delayed fashion over a prolonged period of time.

**Revendications**

**1.** Polymère réticulé, absorbant des liquides aqueux, constitué de monomères partiellement neutralisés, portant des groupes acides monoéthyléniquement insaturés, éventuellement d'autres monomères copolymérisables avec eux, ainsi qu'éventuellement des polymères appropriés comme base de greffage, caractérisé en ce qu'il est préparable par utilisation d'une combinaison d'agent de réticulation/monomère à base de

$$\text{I.} \qquad CH_2=CR^6\text{-}CO(OCHR^3\text{-}CHR^3)_z O\text{-}CH_2\text{-}CR^6=CH_2$$

$$\text{II.} \qquad CH_2=CR^6\text{-}R^5\text{-}(OCHR^3\text{-}CHR^3)_v OR^4$$

$$\text{III.} \qquad R^1\text{-}[O(CHR^3\text{-}CHR^3O)_u\text{-}CO\text{-}R^2]_x,$$

et/ou de di- ou de triallylamine et/ou de bisacrylamides,
   où

$R^1$ : alkyle polyvalent en $C_2$-$C_{10}$,
$R^2$ : alcényle linéaire ou ramifié en $C_2$-$C_{10}$,
$R^3$ : H, $CH_3$, $C_2H_5$,
$R^4$ : H, alkyle linéaire ou ramifié en $C_1$-$C_{10}$,
$R^5$ : CO, $CH_2$,
$R^6$ : H, $CH_3$,
x : 2-6,
u : 0-15,
v: 1-45,
z : 3-20.

**2.** Polymère suivant la revendication 1, caractérisé en ce que les composants I) sont mis en oeuvre avec 0-1,0% en poids, de préférence 0,05-0,6% en poids, par rapport aux monomères, les II) avec 0,1-10% en poids, de préférence 0,5-5% en poids et les III) avec 0,01-1,0% en poids, de préférence 0,05-0,6% en poids.

**3.** Polymère suivant la revendication 1, caractérisé en ce que les agents de réticulation 1) sont mis en oeuvre par rapport aux monomères avec 0,1-0,4% en poids, les II) avec 1,0-3,5% en poids et les III) avec 0,05-0,3% en poids.

**4.** Polymère suivant l'une des revendications 1 à 3, caractérisé en ce que les agents de réticulation selon I sont choisis parmi le groupe des (méth)acrylates d'allylpolyéthylèneglycol, en ce que les monomères selon II sont choisis parmi le groupe des (méth)acrylates de méthylpolyéthylèneglycol et en ce que les agents de réticulation selon III sont choisis parmi le groupe des (méth)acrylates d'éthoxylate de triméthylolpropane, des (méth)acrylates d'éthoxylate de glycérine, des (méth)acrylates d'éthoxylate de pentaérythrite, des di(méth)acrylates de polyéthylèneglycol-$\alpha,\omega$ et de la diallylamine ou triallylamine, du N,N-méthylènebisacrylamide et de l'acide bisacrylamidoacétique.

**5.** Polymère suivant l'une des revendications 1 à 4, caractérisé en ce que les monomères portant des groupes acides insaturés sont choisis parmi le groupe de l'acide acrylique, de l'acide méthacrylique, de l'acide vinylacétique, de l'acide vinylsulfonique, de l'acide méthallylsulfonique et de l'acide 2-acrylamido-2-méthylpropanesulfonique.

6. Polymère suivant l'une des revendications 1 à 5, caractérisé en ce qu'il contient à l'état polymérisé 0 à 40% en poids d'autres comonomères du groupe du (méth)acrylamide, du (méth)acrylonitrile, de la vinylpyrrolidone, de l'acrylate d'hydroxyéthyle et de l'acétamide vinylique.

7. Polymère suivant l'une des revendications 1 à 6, caractérisé en ce qu'il contient jusqu'à 30% en poids de polymères solubles dans l'eau sous la forme de base de greffage, de préférence des polysaccharides et/ou de l'alcool polyvinylique.

8. Polymère suivant l'une des revendications 1 à 7, caractérisé en ce qu'il est réticulé à la surface avec un agent de post-réticulation et en ce que cette post-réticulation est répétée éventuellement à plusieurs reprises.

9. Polymère suivant la revendication 8, caractérisé en ce qu'il est réticulé à la surface avec un agent de post-réticulation du groupe des polyols, des polyépoxydes, des polyamines et des carbonates d'alkylène.

10. Polymère suivant l'une des revendications 8 et 9, caractérisé en ce qu'il présente une rétention d'au moins 30 g/g, une absorption de liquide sous pression (63 g/cm$^2$) d'au moins 20 g/g et une absorption de liquide sous pression (63 g/cm$^2$) selon SDOV d'au moins 18 g/g, ainsi que des fractions solubles après 1 heure d'au maximum 6,5% et après 16 heures d'au maximum 10%, et en ce qu'il possède une absorption contre une pression de 50 g/cm$^2$ pour une surface d'aspiration réduite à 34% (AAP-A$_{34}$) d'au moins 15 g/g et au moins 50% de la valeur de départ de l'absorption contre pression sans réduction de la surface.

11. Polymère suivant l'une des revendications 8, 9 et 10, caractérisé en ce qu'il possède une absorption de liquide sous pression (63 g/cm$^2$) selon SDOV d'au moins 20 g/g, de préférence d'au moins 22 g/g, et une AAP-A$_{34}$ d'au moins 20 g/g.

12. Polymère suivant l'une des revendications 8, 9 et 10, caractérisé en ce qu'il présente une pression de gonflement (20 minutes) d'au moins 600 g, de préférence d'au moins 800 g et très préférablement supérieure à 900 g.

13. Polymère suivant l'une des revendications 8, 9 et 10, caractérisé en ce que les fractions solubles après 16 heures sont au maximum de 8%.

14. Polymère suivant l'une des revendications 8, 9 et 10, caractérisé en ce qu'il présente un absorption de liquide sous pression (63 g/cm$^2$) de plus de 23 g/g et particulièrement avantageusement de plus de 25 g/g.

15. Polymère suivant l'une des revendications 8, 9 et 10, caractérisé en ce qu'il présente une vitesse de l'absorption de liquide inférieure à 40 secondes, de préférence inférieure à 35 secondes et particulièrement avantageusement inférieure à 30 secondes.

16. Procédé de préparation d'un polymère réticulé, absorbant des liquides aqueux, suivant l'une des revendications 1 à 7, caractérisé en ce qu'on polymérise en un hydrogel, selon le procédé d'une polymérisation en solution ou en suspension, une solution aqueuse à base de monomères partiellement neutralisés, portant des groupes acides, insaturés, et d'un mélange d'agents de réticulation à base de

$$\text{I.} \qquad CH_2{=}CR^6{-}CO(OCHR^3{-}CHR^3)_zO{-}CH_2{-}CR^6{=}CH_2$$

$$\text{II.} \qquad CH_2{=}CR^6{-}R^5{-}(OCHR^3{-}CHR^3)_vOR^4$$

$$\text{III.} \qquad R^1{-}[O(CHR^3{-}CHR^3O)_u{-}CO{-}R^2]_x,$$

et/ou de di- ou de triallylamine et/ou de bisacrylamides,
où

R$^1$ : alkyle polyvalent en C$_2$-C$_{10}$,

$R^2$ : alcényle linéaire ou ramifié en $C_2$-$C_{10}$,

$R^3$ : H, $CH_3$, $C_2H_5$,

$R^4$ : H, alkyle linéaire ou ramifié en $C_1$-$C_{10}$,

$R^5$ : CO, $CH_2$,

$R^6$ : H, $CH_3$,

x : 2-6,

u : 0-15,

v : 1-45,

z : 3-20,

avec addition d'agents générateurs de radicaux, et en ce qu'on le fragmente, sèche, broie et tamise.

17. Procédé suivant la revendication 16, caractérisé en ce que les polymères sont traités avec un agent de réticulation de surface et en ce que la réticulation de surface est effectuée à une température élevée.

18. Procédé suivant la revendication 17, caractérisé en ce que le traitement de surface et la réticulation sont effectués à plusieurs reprises.

19. Utilisation des polymères suivant l'une des revendications 1 à 15, comme produits d'absorption pour de l'eau et des liquides aqueux.

20. Utilisation des polymères suivant l'une des revendications 1 à 13, dans des constructions pour l'absorption de liquides corporels.

21. Utilisation des polymères suivant l'une des revendications 1 à 15, comme composants absorbant de l'eau ou des liquides aqueux dans des câbles conducteurs de courant ou de lumière, comme composants dans des matières d'emballage, comme produits d'amendement des sols et pour la culture des plantes.

22. Utilisation des polymères suivant l'une des revendications 1 à 15, dans des articles plans mousse ou non mousse, qui absorbent de l'eau ou des liquides aqueux.

23. Utilisation des polymères suivant l'une des revendications 1 à 15, comme substance de support pour des engrais et d'autres substances actives qui sont à nouveau cédés à l'environnement de manière retardée pendant un intervalle de temps prolongé.